# EUROPÄISCHE PATENTANMELDUNG

(11) **EP 0 566 825 A1**
(43) Veröffentlichungstag der Anmeldung: **27.10.1993**
(21) Anmeldenummer: 93101174.6
(22) Anmeldetag: 27.01.1993
(51) Int. Cl.: A61M 5/145

(54) **Druckinfusionsapparat**

(30) Priorität: 22.04.1992 DE 4213172
(71) Anmelder: B. Braun Melsungen AG, D-34209 Melsungen (DE)
(72) Erfinder: Gerlach, Hans Josef, W-3538 Marsberg (DE); Rath, Dieter,, W-3508 Melsungen (DE); Steger, Jürgen, W-3582 Felsberg (DE); Proell, Dieter, W-3501 Körle (DE)
(74) Vertreter: Selting, Günther, Dipl.-Ing.

(57) **Zusammenfassung**

Der Druckinfusionsapparat weist einen ersten Halter (11) zum Festlegen des Spritzenzylinders (14) einer Spritze (13) und einen linear bewegbaren zweiten Halter (18) auf. An der Kolbenstange (16a) der Spritze (13) greift eine Kolbenbremse (35) an, die bei eingelegtem Spritzenzylinder (14) die Kolbenstange (16a) blockiert, solange die Kolbenstange noch nicht von dem zweiten Halter (18) ergriffen wurde. Dadurch werden unbeabsichtigte Verschiebungen der Kolbenstange (16a) durch Saugwirkung oder durch mechanisches Bewegen vor Infusionsbeginn vermieden.

## Beschreibung

Die Erfindung betrifft einen Druckinfusionsapparat zum Injizieren von Medikamenten oder anderen Flüssigkeiten in den Körper eines Patienten.

Bei Druckinfusionsapparaten zum Ausdrücken einer Spritze wird der Spritzenzylinder an einem ersten Halter festgelegt, während an dem Spritzenkolben ein zweiter Halter angreift, der von einem Motorantrieb linear bewegt wird. Derartige Spritzenpumpen sind beispielsweise bekannt aus DE 31 50 623 C2, DE 34 28 655 C2 und CH 601 763. Zum Einlegen der Spritze muß der rückwärtige zweite Halter von dem Antrieb entkoppelt und manuell in eine Position geschoben werden, die es ermöglicht, die gefüllte Spritze einzulegen. Dabei muß zunächst der Motorantrieb entriegelt, dann der zweite Halter in die gewünschte Position bewegt, die Spritze in die beiden Halter eingelegt und schließlich der Motorantrieb wieder verriegelt werden. Wird der zweite Halter beim Einlegen der Spritze ungenau positioniert, dann wird entweder Luft in die Spritze eingesaugt oder ein Teil der in der Spritze enthaltenen Flüssigkeit ausgedrückt. Das Einsaugen von Luft ist gefährlich, weil diese Luft bei der anschließenden Infusion in den Blutkreislauf des Patienten gelangen kann. Wird Flüssigkeit vor Infusionsbeginn aus der Spritze ausgedrückt, so wird die dem Patienten zuzuführende Menge verfälscht und es geht wertvolles Medikament verloren. Ferner kann es vorkommen, daß durch einen Unterdruck oder Überdruck in Folge eines hydrostatischen Gefälles zwischen Patient und Spritze Flüssigkeit aus der Spritze heraus unkontrolliert angesaugt wird, wobei der Spritzenkolben nachgezogen wird, wenn er noch nicht mit dem zweiten Halter gekoppelt ist. Auch hierbei ergibt sich ein unkontrollierter Medikamentenstoß außerhalb der vorgesehenen Infusionsrate.

Der Erfindung liegt die Aufgabe zugrunde, einen Druckinfusionsapparat zu schaffen, der unkontrollierte Medikamentenzugabe vor Infusionsbeginn sowie das Ansaugen von Luft in die Spritze sicher vermeidet.

Die Lösung dieser Aufgabe erfolgt erfindungsgemäß mit den im Patentanspruch 1 angegebenen Merkmalen.

Bei dem erfindungsgemäßen Druckinfusionsapparat wird der Spritzenkolben vor Infusionsbeginn von einer Kolbenbremse festgehalten, die nach dem Einsetzen des Spritzenzylinders in den ersten Halter wirksam ist und den Kolben mechanisch blockiert. Die Kolbenbremse ist relativ zu dem ersten Halter ortsfest angeordnet und sie bewirkt zusammen mit dem ersten Halter eine kraftschlüssige und formschlüssige Verbindung zwischen Spritzenzylinder und Spritzenkolben. Daher wird nach dem Einlegen des Spritzenzylinders in den ersten Halter der Kolben verriegelt, so daß er nicht verschoben werden kann. Der zweite Halter kann dann an das rückwärtige Ende der Kolbenstange heranbewegt und mit dieser gekoppelt werden, ohne daß die Gefahr der unbeabsichtigten Verschiebung der Kolbenstange besteht. Auch wenn der Patientenkörper eine Sogwirkung oder Druckwirkung auf die Spritze ausübt, wird der Spritzenkolben durch die Kolbenbremse festgehalten, so daß er nicht nachgibt und die Spritze keine Flüssigkeit abgibt, solange die Kolbenbremse geschlossen ist.

Vor Beginn des Infusionsvorganges wird die Kolbenbremse gelöst oder entriegelt, so daß der Kolben nunmehr durch den angetriebenen zweiten Halter zur Durchführung der Infusion vorgeschoben werden kann.

In vorteilhafter Weiterbildung der Erfindung enthält der zweite Halter einen das Einlegen der Kolbenstange feststellenden Sensor, dessen Signal die Kolbenbremse öffnet. Der Sensor stellt fest, wann die Kopplung der Kolbenstange mit dem zweiten Halter erfolgt und die Bremswirkung nicht mehr erforderlich ist. Zweckmäßigerweise ist der zweite Halter mit einer Verriegelungsvorrichtung versehen, die das rückwärtige Ende der Kolbenstange an dem zweiten Halter fixiert und keine Vorwärtsbewegung der Kolbenstange ohne den zweiten Halter zuläßt. Auf diese Weise wird vermieden, daß der Kolben durch eine während der Infusion auftretende Sogwirkung aus dem zweiten Halter herausgleitet.

Die Kolbenbremse kann seitlich gegen die Kolbenstange drücken. Dabei ist es zweckmäßig, ein Gegenlager vorzusehen, das die Kolbenstange abstützt. Dieses Gegenlager ist von einer inaktiven Position in eine Abstützposition bewegbar und es ist ein Sensor vorgesehen, der das Einnehmen der Abstützposition feststellt und dessen Signal die Kolbenbremse betätigt. Die Kolbenbremse wird also erst dann betätigt, wenn sich das Gegenlager in seiner Abstützposition befindet und wenn das rückwärtige Ende der Kolbenstange noch nicht an dem zweiten Halter angreift.

Im folgenden wird unter Bezugnahme auf die Zeichnungen ein Ausführungsbeispiel der Erfindung näher erläutert.

Es zeigen:

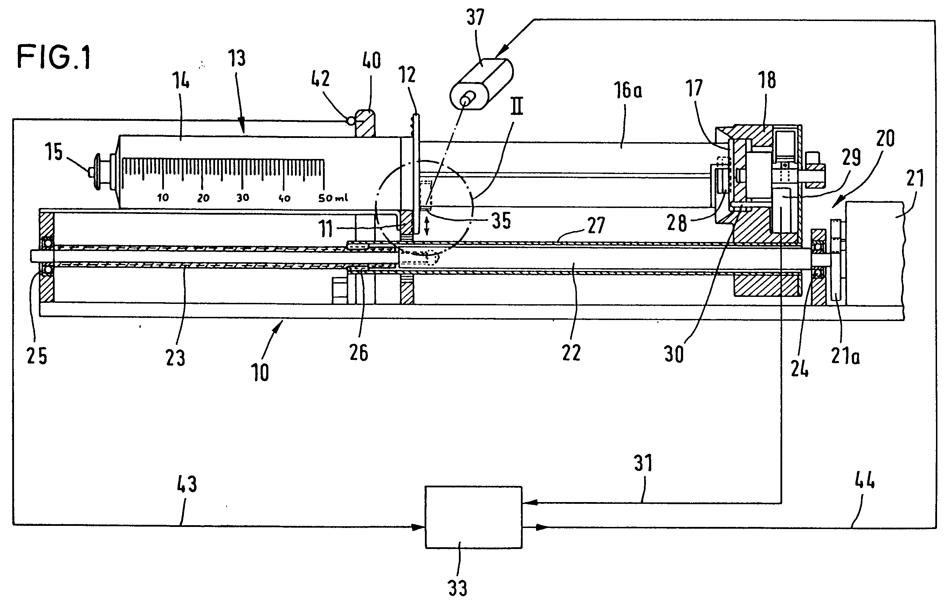

- Fig. 1: eine Draufsicht des Druckinfusionsapparates, teilweise geschnitten, mit eingelegter Spritze und

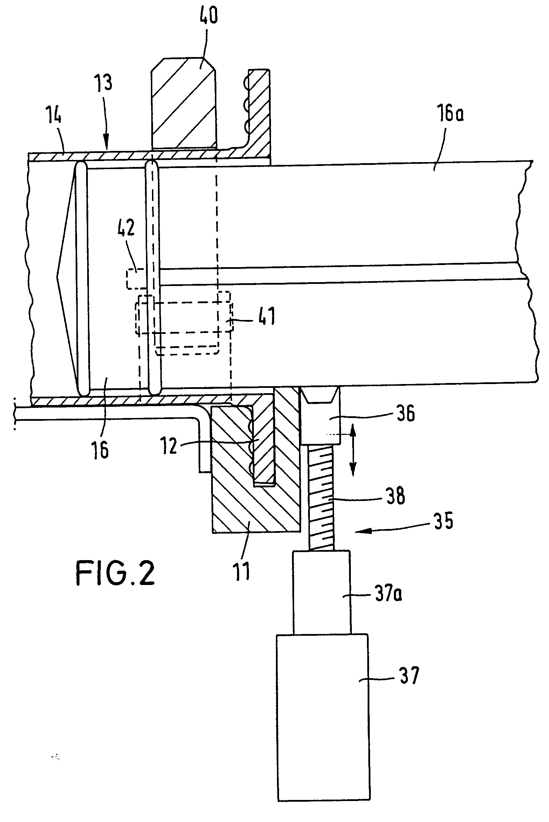

- Fig. 2: eine vergrößerte Darstellung der Einzelheit II aus Fig. 1 im Schnitt.

Der Druckinfusionsapparat weist ein Gehäuse 10 auf, das in der Zeichnung durch einen horizontalen Balken angedeutet ist, an dem die einzelnen Komponenten befestigt sind. Am Gehäuse 10 ist ein erster Halter 11 angeordnet, an dem die Halteplatte 12 einer Spritze 13 festgelegt werden kann. Die Halteplatte 12 ist am rückwärtigen Ende des Spritzenzylinders 14 angebracht, der an seinem vorderen Ende einen Auslaßstutzen 15 für den Anschluß einer zum Patienten führenden Flüssigkeitsleitung aufweist. Der erste Halter 11 hält die Spritze 13 in definierter axialer Ausrichtung fest. In dem Spritzenzylinder 14 befindet sich der Spritzenkolben 16, der mit einer Kolbenstange 16a verbunden ist, welche aus dem rückwärtigen Ende des Spritzenzylinders herausragt. Am rückwärtigen Ende der Kolbenstange 16 ist eine querverlaufende Betätigungsplatte 17 vorgesehen.

Die Betätigungsplatte 17 ist beim Betrieb des Druckinfusionsapparates in den zweiten Halter 18 eingesetzt, der linear in Richtung auf den ersten Halter bewegt werden kann, um den Spritzenkolben 16 im Spritzenzylinder 14 vorzuschieben und die Flüssigkeit aus dem Auslaßstutzen 15 herauszudrücken.

Der zweite Halter 18 wird von einem Motorantrieb 20 angetrieben. Dieser Antrieb weist einen am Gehäuse 10 befestigten Motor 21 auf, der über ein Getriebe 21a eine Ausgangswelle 22 antreibt, die mit einer Schraubenspindel 23 fest verbunden ist. Die Ausgangswelle 22 und die Schraubenspindel 23 sind in Lagern 24,25 im Gehäuse gelagert. Auf dem Schraubengewinde der Schraubenspindel sitzt eine Spindelmutter 26, die über ein die Ausgangswelle 22 umgebendes Rohr 27 mit dem zweiten Halter 18 fest verbunden ist. Der Motor 21 treibt über das Untersetzgetriebe 21a die Ausgangswelle 21 an, wodurch die Schraubenspindel 23 gedreht wird. Dadurch wird die Spindelmutter 26, die gegen Drehung festgehalten ist, entlang der Schraubenspindel 23 linear bewegt. Diese Linearbewegung wird durch das Rohr 27 auf den zweiten Halter 18 übertragen.

Der zweite Halter 18 weist eine Verriegelungsvorrichtung 28 auf, die die Betätigungsplatte 17 umgreift, so daß die Betätigungsplatte nicht aus dem zweiten Halter herausgezogen werden kann. Ferner enthält der zweite Halter 18 einen Sensor 29 mit einem Taststift 30, der das Vorhandensein der Betätigungsplatte 17 der Spritze in dem Halter 18 feststellt. Dieser Sensor 29 ist über eine Leitung 31 mit einer Steuereinrichtung 33 verbunden. Die Steuereinrichtung 33 ist z.B. ein Mikroprozessor, der den Funktionsablauf des Druckinfusionsapparates, einschließlich des Betriebes des Motorantriebs 20, steuert.

In der Nähe des ortsfesten ersten Halters 11 ist an dem Gehäuse 10 oder an dem damit fest verbundenen ersten Halter 11 eine Kolbenbremse 35 angebracht. Diese Kolbenbremse weist ein Blockierelement 36 auf, das radial gegen die Kolbenstange 16a gedrückt werden kann. Das Blockierelement 36 wird von einer Antriebseinrichtung angetrieben, die einen Motor 37 mit Getriebe 37a und eine Spindel 38 aufweist, an der das Blockierelement 36 angebracht ist. Dieser Antrieb ist ortsfest am Gehäuse 10 angebracht. Durch Betätigung des Motors 37 wird das Blockierelement 36 in Richtung auf die Kolbenstange 16a bewegt bis das Blockierteil 36 fest gegen die Kolbenstange 16a drückt und deren Längsbewegung blockiert.

An der der Kolbenbremse 35 abgewandten Seite der Spritze 13 ist ein Gegenlager 40 vorgesehen, das den Spritzenzylinder gegen die Kraft der Kolbenbremse 35 abstützt. Dieses Gegenlager 40 ist als Haltebügel ausgebildet, der von einer inaktiven Position in eine Abstützposition schwenkbar ist. Das Gegenlager 40 ist um ein fest am Gehäuse 10 angebrachtes Gelenk 41 herum schwenkbar. Ein Sensor 42 spricht an, wenn das Gegenlager 40 seine Abstützposition eingenommen hat. Dieser Sensor 42 ist über eine Leitung 43 mit der Steuereinrichtung 33 verbunden. Die Steuereinrichtung 33 steuert über eine Leitung 44 den Motor 37 der Kolbenbremse 35.

Wenn eine Spritze 13 in den Druckinfusionsapparat eingesetzt wird, befindet sich der zweite Halter 18 in einer rückwärtigen Position, in der er das Einsetzen der Spritze nicht behindert. Das Gegenlager 40 ist im Öffnungszustand. Der Spritzenzylinder 14 wird mit seiner Halteplatte 12 in den ersten Halter 11 eingesetzt. Die Steuereinrichtung stellt fest, daß der Sensor 29 das Vorhandensein der Kolbenstange in dem Halter 18 noch nicht erkannt hat und daß das Gegenlager 40 noch geöffnet ist. Das Gegenlager 40 wird dann manuell geschlossen und dies wird der Steuereinrichtung 33 über Leitung 43 mitgeteilt. Die Steuereinrichtung 33 stellt fest, daß das Gegenlager 40 geschlossen ist, daß die Betätigungsplatte 17 aber noch nicht im zweiten Halter 18 aufgenommen wurde. Daraufhin betätigt die Steuereinrichtung 33 die Kolbenbremse 35, deren Blockierelement 36 vorgeschoben wird und die Kolbenstange 16 blockiert. Danach kann manuell oder durch Motorantrieb der zweite Halter 18 vorgeschoben werden, bis er gegen die Betätigungsplatte 17 stößt und diese mit seiner Verriegelungsvorrichtung 28 festhält. Dieser Zustand wird von dem Sensor 29 erkannt und der Steuereinrichtung 33 mitgeteilt. Die Steuereinrichtung löst daraufhin die Kolbenbremse 35. Der Druckinfusionsapparat ist nunmehr für den Infusionsvorgang bereit.

Abweichend von dem beschriebenen Ausführungsbeispiel ist es auch möglich, eine manuell zu betätigende Kolbenbremse 35 vorzusehen, die nach dem Einlegen des Spritzenzylinders 14 in den ersten Halter 11 verriegelt wird und die entriegelt wird, nachdem der zweite Halter 18 die Betätigungsplatte 17 aufgenommen hat.

## Patentansprüche

1. Druckinfusionsapparat mit einem ersten Halter (11) zum Festlegen des Zylinders (14) einer Spritze (13), einem zweiten Halter (18) zum Angreifen am rückwärtigen Ende der Kolbenstange (16a) der Spritze (13) und einem Motorantrieb (20) zum linearen Bewegen des zweiten Halters (18) relativ zu dem ersten Halter (11),
**dadurch gekennzeichnet,**
daß eine an der Kolbenstange (16a) der in den ersten Halter (11) eingesetzten Spritze (13) angreifende Kolbenbremse (35) zum Festhalten der Kolbenstange (16a) vor Infusionsbeginn vorgesehen ist.

2. Druckinfusionsapparat nach Anspruch 1, dadurch gekennzeichnet, daß der zweite Halter (18) einen das Einlegen der Kolbenstange (16a) feststellenden Sensor (29) enthält, dessen Signal die Kolbenbremse (35) öffnet.

3. Druckinfusionsapparat nach Anspruch 1 oder 2, dadurch gekennzeichnet, daß an der der Kolbenbremse (35) gegenüberliegenden Seite ein Gegenlager (40) zum Abstützen des Spritzenzylinders (14) angeordnet ist.

4. Druckinfusionsapparat nach Anspruch 3, dadurch gekennzeichnet, daß das Gegenlager (40) von einer inaktiven Position in eine Abstützposition bewegbar ist und daß ein das Einnehmen der Abstützposition feststellender Sensor (42) vorgesehen ist, dessen Signal die Kolbenbremse (35) betätigt.
